# EUROPEAN PATENT SPECIFICATION

(11) **EP 0 473 252 B1**
(45) Date of publication and mention of the grant of the patent: **05.03.1997**
(21) Application number: 91302820.5
(22) Date of filing: 28.03.1991
(51) Int. Cl.: A61K 31/135

(54) **Use of L-deprenyl for retarding deterioration due to aging in dogs**
Verwendung des L-Deprenyls zur Verzögerung der altersbedingten Zerstörung des Gedächtnis bei Hunden
Utilisation de L-déprényl pour ralentir la détérioration due au vieillissement aux chiens

(30) Priority: 31.08.1990 US 576011; 18.01.1991 US 643452
(43) Date of publication of application: 04.03.1992
(60) Divisional application: 96103701.7
(73) Proprietor: DEPRENYL ANIMAL HEALTH, INC., Overland Park, Kansas 66210 (US)
(72) Inventor: Milgram, Norton W., Scarborough Campus, Scarborough, Ontario, MIC 1A4 (CA); Stevens, David R., Lawrence, Kansas 66046 (US); Ivy, Gwendolyn O., Scarborough Campus, Scarborough, Ontario, MIC 1A4 (CA)
(74) Representative: Thomson, Paul Anthony

(56) References cited:
- WO-A-90/01928
- RESEARCH COMMUNICATIONS IN CHEMICAL PATHOLOGY AND PHARMACOLOGY, vol. 52, no. 2, 1986, pages 207-216; M. SENJO et al.: "Decreased monoamine oxidase activity in stroke-prone spontaneously hypertensive rat kidneys"
- BIOL. PSYCHIATRY, vol. 20, no. 5, 1985, pages 558-565; M.R. LIEBOWITZ et al.: "Biochemical effects of L-deprenyl in atypical depressives"
- JAPANESE JOURNAL OF PHARMACOLOGY, vol. 29, suppl., page 178P, abstract no. P-53; H. SATO et al.: "Effect of oxygen concentration no MAO activity in dog kidney"
- BR. J. PHARMACOL., vol. 86, no. 4, 1985, pages 877-888; F. GARCHA et al.: "[14 C]-beta-phenethylamine, its distribution after administration by various routes to cats, and the effects of monoamine oxidase inhibitors"
- XENOBIOTICA, vol. 17, no. 8, 1987, pages 957-963; T. YOSHIDA et al.: "Hepatic and extrahepatic metabolism of deprenyl, a selective monoamine oxidase (MAO) B inhibitor, of amphetamines in rats: sex and strain differences"
- PSYCHOPHARMACOLOGY. vol. 91, no. 4, 1987, pages 489-495; P.N. TARIOT et al.: "Cognitive effects of L-deprenyl in Alzheimer's disease"
- ACTA NEUROL. SCAND, vol. 95, suppl., 1983, pages 135-144; R. PORTIN et al.: "The effect of deprenyl (selegiline) on cognition and emotion in parkinsonian patients undergoing long-term levodopa treatment"
- CLINICAL THERAPEUTICS, vol. 12, no. 5, 1990, pages 376-384; A. FALSAPERIA et al.: "Selegiline versus oxiracetam in patients with Alzheimer-type dementia"
- ACTA NEUROL. SCAND., vol. 126, 1989, pages 83-91; J. KNOLL: "The pharmacology of selegiline ((-)deprenyl). New aspects"
- FUNCTIONAL NEUROLOGY, vol. 1, no. 2, April-June 1986, pages 165-174; F. DRAGO et al.: "Behavioral effects of deprenyl in aged rats"
- ACTA NEUROL. SCAND., vol. 80, no. 126, 1989, pages 103-111; E.H. HEINONEN et al.: "Selegiline in the treatment of Parkinson's disease"
- MECHANISMS OF AGEING AND DEVELOPMENT, vol. 30, no. 2, 1985, pages 109-122; J. KNOLL: "The facilitation of dopaminergic activity in the aged brain by (- )deprenyl. A proposal for a strategy to improve the quality of life in senescence"
- Comprehensive Psychiatry, vol. 30, no. 2, 1986, p. 135-138
- J. Clin. Psychiatry, vol. 48, no 9, Suppl. 1987, p. 20-23
- Psychopharmacology bulletin, vol. 23, no. 1, 1987, p.26-29
- J Clin. Psychopharmacology, vol. 9, no. 3, 1989, p. 180-185
- Goodman and Gilmans The Pharmacological Basis of Therapeutics, eighth edition, p. 466-479

## Description

L-deprenyl is a selective monoamine oxidase B (MA0-B) inhibitor, which is widely used as an adjunct in the treatment of Parkinson's disease. While it's most common usage is for the treatment of Parkinson's disease, L-deprenyl was originally developed as an antidepressant agent. Recent testing has indicated that L-deprenyl may have some effect on increasing sexual response in aging animals, and also may have some effect, at least in rats in increasing the natural life span. However, to date L-deprenyl has only been medically approved by regulatory agencies for use as an adjunctive treatment for Parkinson's disease.

The search for new lines of medication to improve the quality of life in senescence ever continues. This becomes especially important in modern-day society, especially in developed countries, where the proportion of citizens over 65 years of age continues to increase. In sum, the quality of life has become increasingly important in older years, as people continue to experience longer life expectancy.

There is, therefore, a continuing and real need for the development of medications which retard the normal deterioration of certain bodily functions.

According to the present invention there is provided the use of the compound L-deprenyl, or a pharmaceutically acceptable form thereof, for the manufacture of a medicament for retarding the normal age-dependent deterioration of the cognitive process of dogs.

Also according to the present invention there is provided the use of the compound L-deprenyl, or a pharmaceutically acceptable form thereof, for manufacture of a medicament for retarding the age-dependent deterioration of exploratory behaviour of dogs.

Preferably the L-deprenyl used is the levorotary optical isomer.

Further preferably, the L-deprenyl is the hydrochloride addition salt form thereof.

Further preferably, the L-deprenyl or the pharmaceutically acceptable form thereof, is used at levels from 0.1 mg/kg of body weight to 5.0 mg/kg of body weight.

### DETAILED DESCRIPTION OF THE INVENTION

As earlier stated, the compound that is useful for the method or protocol of the present invention is a known compound, L-deprenyl. L-deprenyl has the formula (-)-N-α-dimethyl-N-2-propynylbenzene-ethanamine. It can be illustrated by the following graphic formula:

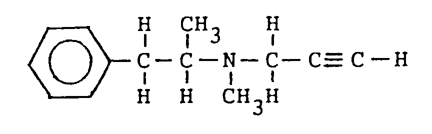

L-Deprenyl also is at times referred to as (-)deprenyl to illustrate that it is a levorotary isomer which is the active form for treatment of Parkinson's disease. Typically, it is provided in a pharmaceutically acceptable salt form thereof such as the hydrochloride salt.

As used here, pharmaceutically acceptable salt form thereof, means the following. Acceptable for use in the pharmaceutical or veterinary art, being nontoxic or otherwise not pharmaceutically or veterinary unacceptable. "Acceptable salt form thereof" means salts formed with inorganic acids such as hydrochloric acid, hydrobromic acid, sulfuric acid, nitric acid, phosphoric acid, and the like, and as well organic acids such as acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, etc.

Administration of the prophylactively active compound L-deprenyl to achieve physiological results of the present invention can be via any of the accepted modes of administration for systemically active substances. These methods include oral, parenteral, and otherwise systemic, aerosol, and topical forms, as well as sustained release systems, etc.

The compositions of the present invention may be any of those known in the pharmaceutical and veterinary arts which are suitable for the method of administration and dosage required in any particular circumstance. In the case of both pharmaceutical and veterinary applications, such compositions may include tablets, pills, capsules, powders, aerosols, suppositories, skin patches, parenterals, and oral liquids including oil aqueous suspensions, solutions and emulsions. It may include long acting injectables and sustained release devices.

When the dosage is in solid form, solid pharmaceutical carriers such as starch, sugar, talc, mannitol, povidone, magnesium stearate, and the like may be used to form powders. Lactose and mannose are the preferred solid carrier. The powders may be used as such for direct administration to a patient or, instead, the powders may be added to suitable foods and liquids, including water, to facilitate administration.

The powders also may be used to make tablets, or to fill gelatin capsules. Suitable lubricants like magnesium stearate, binders such as gelatin, and disintegrating agents like sodium carbonate in combination with citric acid may be used to form the tablets.

Unit dosage forms such as tablets and capsules may contain any suitable predetermined amount of L-deprenyl, advisably as a nontoxic acid addition salt, and may be administered one or more at a time at regular intervals as later described. Such unit dosage forms, however, should with a broad range guideline contain a concentration of 0.1 mg/kg to 5.0 mg/kg of one or more forms of the active L-deprenyl.

A typical tablet for the specified uses mentioned herein in a 25 kg dog may have the composition:

| | Mg. |
|---|---|
| 1. L-deprenyl | 25 |
| 2. Mannitol | 100 |
| 3. Stearic acid | 3 |

A granulation is made from the mannitol. The other ingredients are added to the dry granulation and then the tablets are punched.

Another tablet may have the composition:

| | Mg. |
|---|---|
| 1. L-deprenyl | 25 |
| 2. Starch U.S.P. | 57 |
| 3. Lactose U.S.P. | 73 |
| 4. Talc U.S.P. | 9 |
| 5. Stearic acid | 6 |

Powders 1, 2 and 3 are slugged, then granulated, mixed with 4 and 5, and tableted.

Capsules may be prepared by filling No. 3 hard gelatin capsules with the following ingredients, thoroughly mixed:

| | Mg. |
|---|---|
| 1. L-deprenyl | 25 |
| 2. Lactose U.S.P. | 200 |
| 3. Starch U.S.P. | 16 |
| 4. Talc U.S.P. | 8 |

As earlier expressed, physiological functions affected by the treatment herein with L-deprenyl are necessarily dosage dependent. Put another way, like most drugs, L-deprenyl has diverse physiological effects depending upon the dose administered. Unless the dose administered is within the levels set forth herein, the desired effects on thyroid function, adrenal function, immune function and body weight are not achieved without adverse effects.

Aging has long been thought of as being associated with decreased adaptiveness to stress (Selye and Tuchweber, "Stress in relation to aging and disease", Hypothalamus, Pituitary and Aging, pp. 557-573, 1976). For example, basal body temperature is similar in both young and aged humans, but aged individuals are less able to regulate their temperature when heat or cold challenged (Shock, "Systems integration", In: Handbook of the Biology of Aging, New York, 1977). There is also some evidence that stress itself can accelerate certain biomarkers of aging (Curtis, "Biological mechanisms underlying the aging process", Science 141, 686, 1963; Pare, "The effect of chronic environmental stress on premature aging in the rat", J. Gerontol, 20, 78, 1965). Studies by Sapolsky's and Landfield's groups support the notion of a "glucocorticoid cascade" (Sapolsky et al., "The neuroendocrinology of stress and aging: The glucocorticoid cascade hypothesis", Endocrine Reviews 7, 284-301, 1986), in which both aging and stress lead to a decreased ability to terminate the secretion of adrenocortical stress hormones (glucocorticoids) at the end of a stress response. Cumulative exposure to glucocorticoids causes degenerative changes in the hippocampus, a brain region which normally inhibits glucocorticoid release. The degeneration, in turn, further decreases the brain's ability to terminate the stress response, thus leading to more damage and effectively forming a feed-forward cascade with potentially serious pathophysiological consequences in the old individual. For example, an overabundance of glucocorticoids, as seen in prolonged stress and in Cushing's syndrome, can cause myopathy, steroid diabetes, hypertension, immunosuppression, infertility and inhibition of growth (Munck et al., "Physiological functions of glucocorticoids during stress and their relation to pharmacological actions", Endocrine Review 5, 25-49, 1984; Krieger, "Cushing's syndrome", Monographs on Endocrinology 22, 1-122, 1982).

Although the thyroid gland may have sufficient capacity to maintain its principal hormones, T3 & T4, within normal ranges during the senescent phase of life, there is some suggestion that age-related intermittent thyroid deficiency may arise as a consequence of stress, or that the thyroid hormones are potentially less biologically effective (Morse, D. R. Aging: Causes and Control. Internat. J. Psychosomat. 35: 12-42, 1988). Likewise, thyroid stimulating hormone (TSH), secreted from the pituitary gland, functions within the negative feedback control mechanism associated with the hypothalamus, portions of which operate under the trophic influences of the dopaminergic neurons. With age, the nigrostriatal dopamine secreting neurons degenerate, with the possible consequence of perturbation of the hypothalamic-hypophyseal system, again predicting a decrease of thyroid gland control during senescence. Further, hypothyroidism is often associated with aberrant immune function, often termed immune-mediated or autoimmune hypothyroidism. This, too, may be a consequence of aging (see below).

Immune reactions involve the coordinated efforts of three lymphocyte subpopulations: T and B lymphocytes and antigen presenting cells. Substantial evidence exists that immune function declines with age and that impaired T-cell function may be largely responsible for the decline. While some changes in B lymphocyte function have been reported, it is difficult to dissociate these effects from concomittant changes in T-Cells. Also, little or no evidence supports changes in antigen presenting cells with age.

On the other hand, a number of investigators have shown a substantial decline in most measures of T-cell function with age. The most studied of the immune parameters is lymphocyte proliferation. The proliferative capacity of both T- and B-cells has been found to decline substantially with age (Morgan et al., "The immune response in aged C57BL/6 mice. I. Assessment of lesions in the B-cell and T-cell compartments of aged mice utilizing the Fc fragment-mediated polyclonal antibody response", Cellular Immunology 63, 16-27, 1981; Abraham et al., "Reduced in vitro response to concanavalin A and lipopolysaccharide in senescent mice: A function of reduced number of responding cells", European Journal of Immunology 7, 301-304, 1977; Hefton et al., "Immunologic studies of aging. V. Impaired proliferation of PHA responsive human lymphocytes in culture", Journal of Immunology 125, 1007-1010, 1980). These studies utilize splenocytes which are mitogenically challenged with phytohemaglutin (PHA) or concanavalin-A in vitro in the presence of 3H-thymidine or bromodeoxyuridine (BrdU). Thymidine or BrdU uptake by the cells is then measured and reflects the number of cells entering mitosis. Also, upon activation by mitogens or antigens, T-cells secrete a number of antigen-non-specific growth and maturation factors, collectively termed lymphokines. One of these, Interleukin-2 (IL-2), seems to be required for T-cell division and plays a role in B-cell growth as well. T-cells from old mice and humans have been found to secrete diminished amounts of IL-2 when triggered by mitogens, alloantigens or foreign antigens (Gillis et al., "Immunological studies of aging. Decreased production of and response to T cell growth factor by lymphocytes from aged humans", Journal of Clinical Investigation 67, 937-942, 1981; Miller and Stutman, "Decline, in aging mice, of the anti-TNP cytotoxic T cell response attributable to loss of Lyt-2, IL-2 producing helper cell function", European Journal of Immunology 11, 751-756, 1981; Nagel et al., "Decreased proliferation interleukin 2 synthesis, and interleukin 2 receptor expression are accompanied by decreased mRNA expression in phytohemag glutinin-stimulated cells from elderly donors", Journal of Clinical Investigation 81, 1096-1102, 1988; Thomas and Weigle, "Partial restoration of Con A-induced proliferation, IL-2 receptor expression, and IL-2 synthesis in aged murine lymphocytes by phorbol myristate acetate and ionomycin", Cellular Immunology 114, 1-11, 1988). Further, several studies have shown that aging leads not only to poor production of IL-2, but also to diminished responsiveness to this growth factor. Thus, addition of exogenous IL-2 leads to only a partial restoration of function in immune cells from old animals (Gillis et al., "Immunological studies of aging. Decreased production of and response to T cell growth factor by lymphocytes from aged humans", Journal of Clinical Investigation 67, 937-942, 1981; Gilman et al., "T lymphocytes of young and aged rats. II. Functional defects and the role of interleukin-2", Journal of Immunology 128, 644-650, 1982; Gottosman et al., "Proliferative and cytotoxic immune functions in aging mice. III. Exogenous interleukin-2 rich supernatant only partially restores alloreactivity in vitro", Mechanisms of Ageing and Development 31, 103-113, 1985). Also, the number of T-cells able to express IL-2 receptors upon stimulation by a mitogenic agent declines with age in both humans and mice (Negoro et al., "Mechanisms of age-related decline in antigenspecific T cell proliferative response: IL-2 receptor expression and recombinant IL-2 induced proliferative response of purified TAC-positive T cells", Mechanisms of Ageing and Development 36, 223-241, 1986; Vie and Miller, "Decline, with age, in the proportion of mouse T cells that express IL-2 receptors after mitogen stimulation", Mechanisms of Ageing and Development 33, 313-322, 1986). Aging, therefore, leads to a decline in both production of and response to IL-2.

Growth hormone (GH) levels are known to fall in both male and female rats with age. In female rats, a 50% decrease in GH secretion can be demonstrated at 11 months and may account for the stasis in body growth which occurs at about this time (Takahasi, S., Goya, R., and Meites, J., "Growth hormone secretory patterns in young, middle aged and old female rats", Neuroendocrinology 46, 137-142, 1987). Aging male rats show a marked decrease in GH secretion, but the time course of the decrease is not known (Sonntag et al., "Decreased pulsatile release of growth hormone in old male rats", Endocrinology 107, 1875-1879, 1980). Somatomedin (SM) levels in the circulation, which are under GH control, also fall markedly with age. These decreases in GH and SM secretion are of primary importance as they may cause the age related decrease in protein synthesis, which may have far reaching implication for the ability of aged organisms to maintain body composition and homeostasis.

The total amount of protein in the human body decreases with age after about 40 years, due largely to a loss of skeletal muscle mass (Young et al., "Human aging: protein and amino acid requirements", Nutritional Approaches to Aging Research, 47, CRC Press, 1982). This decrease is likely due to decreases in protein synthesis which are known to occur in a variety of organisms with age. Body water also decreases with age in humans, beginning at birth and proceeding at a fairly constant rate throughout life (Shock et al., "Age differences in the water content of the body as related to basal oxygen consumption in males", J. Gerontol 18, 1, 1963; Timiras, Developmental Physiology and Aging, Macmillan, New York, 1972). Total body fat increases with age in humans, until at least the fifth decade of life; data on humans of advanced age are conflicting (Meneely et al., "Analysis of factors affecting body composition determined from potassium content in 915 normal subjects", Ann. N.Y. Acad. Sci. 110, 271, 1963; Forbes and Reina, "Adult lean body mass with age: some longitudinal observations", Metabolism 19, 653, 1970; Myhre and Kessler, "Body density and potassium 40 measurements of body composition as related to age", J. Appl. Physiol. 21, 1251, 1966; Novak, "Aging, total body potassium, fat free mass in males and females between the ages of 18 and 85 years", J. Gerontol 27, 438, 1972). In contrast, in Fischer 344 rats, total body fat increases until about 75% of their lifespan, but decreases after that (Bertrand et al., "Changes in adipose mass and cellularity throughout the adult life of rats fed ad libitum or a life-prolonging restricted diet", J. Gerontol. 35, 827, 1980). Specific age related changes in serum lipids are difficult to establish because of their dependence on whole body fat, exercise and diet, and available data are conflicting. Similarly, changes in serum lipoprotein levels with age are influenced by a number of factors and do not appear to be age- so much as health and habit-related.

Serum albumin levels in humans appear to be unaffected by age. In rats, albumin levels appear to depend on strain and on whether chronic nephropathy is common, as in Fischer 344 rats, which have nephropathy and concomittant decreases in serum albumin levels (Heiss et al., "The epidemiology of plasma HDL-cholesterol levels: The Lipid Research Clinic Prevalence Study, Summary", Circulation 62 (Suppl. 4), 116, 1980). Connective tissue changes are common with age. Collagen, the major protein component, becomes more insoluble, rigid, and resistant to enzyme digestion with age (Hamlin and Kohn, "Evidence for progressive age-related structural changes in post-mature human collagen", Biochim. Biophys. Acta 236, 458, 1971; Everitt and Delbridge, "Two faces of collagen ageing in the tail tendon of hypophysectomized rats", Exp. Gerontol. 7, 45, 1972). However, in young and aging male and female Wistar rats, no changes in collagen were found with age (Porta et al., "Effects of the type of dietary fat at two levels of vitamin E in Wistar male rats during development and aging. II. Biochemical and morphometric parameters of the brain", Mech. Ageing Dev. 13, 319, 1980; "Effects of the type of dietary fat at two levels of vitamin E in Wistar male rats during development and aging. III. Biochemical and morphometric parameters of the liver", Mech. Ageing Dev. 15, 297, 1981; "Effects of the type of dietary fat at two levels of vitamin E in Wistar male rats during development and aging. IV. Biochemical and morphometric parameters of the heart", Mech. Ageing Dev. 18, 159, 1982). Thus, reliable age-related changes in body composition across species appear to be restricted to decreases in total body protein and water content.

From the above descriptions, it can be seen that age can adversely impact each of the functions of the adrenal gland, the thyroid, the immune system and body composition as mammals get older. Here, providing that the dosages hereinafter described are used, these functions can be maintained for longer periods of time in older animals at normal levels.

The term "mammal" as used herein includes without limitation humans and domesticated animals such as cattle, horses, swine, sheep, dogs, cats, goats and the like. The tests hereinafter shown in the examples are particularly illustrative for dogs, particularly beagles, but indicate usage for other domesticated pets including cats. The treatment may even work for birds or fish.

Needless to say, the natural enjoyment of these pets would be significantly increased in their older age if one could retard the decrease in the enumerated physiological functions. These natural changes alter the animal's personality such that the human owner has less enjoyment from the animal.

In accordance with the present invention, it is illustrated that each of the above enumerated physiological functions can be maintained for longer periods if the animal is treated periodically with small but prophylactically effective doses of L-deprenyl.

As hereinafter explained, the dosage regimen to achieve these desirable results differs considerably from the dosage regimen used in treating Parkinson's disease. At the highest doses recommended for uses disclosed herein, the levels are less than one-half the amount used for treating Parkinson's disease. In particular, the dosage regimen of the present invention shows usage at levels from about 0.1 mg/kg of body weight up to about 5.0 mg/kg of body weight from 1 to 5 times weekly. Most preferably the dosage level is 1-2 mg/kg of body weight given twice weekly, starting in mid-life. Of course it would be known to those in the art that sustained release systems can be used to provide less frequent administration to achieve the required dosage level.

It is not known precisely why the use of L-deprenyl at the dosage levels and periodicity expressed herein achieves these results. While not wishing to be bound by any theory of operation of the present process, it is believed that rather than solely affecting brain chemistry, there may be a direct effect on the organs involved. The effect may be hypotensive. As explained in the examples below, analysis of serum chemistry perhaps suggests that the L-deprenyl treatment may have a direct effect on specific organ function. With regard to the maintenance of body composition, it is simply not known by what mechanism the compound works, except to say that it is critically important that the dosage be at levels expressed herein rather than at Parkinson's disease levels, otherwise adverse effects may be achieved, particularly in dogs.

The following examples, generally prophetic in nature, are presented to provide the illustrative evidence of the likely results for testing with beagles for maintenance of normal thyroid function, normal adrenal function, normal immune system function, and for normal body composition.

Male and female beagles are treated with L-deprenyl or vehicle beginning at four to seven years of age (GH) (SM) and protein synthesis levels are measured two to five years later. L-deprenyl treated dogs are found to maintain significantly higher levels of each of these than do control dogs, thus appearing more like younger dogs. The dose level is 0.1 to 5 mg/kg for a time of months to years, with dosing being from a composition comprising tablets given orally. The frequency of dosing is from one to five times weekly. This example relates to maintenance of body composition.

Male and female beagles are treated with L-deprenyl or vehicle beginning at four to seven years of age. Two to five years later, animals are evaluated for ability to terminate the stress response to 1) prolonged loud noise, 2) cold exposure and 3) immobilization, by measurement of induced vs basal levels of corticosterone in the bloodstream over the subsequent 24 hours. The L-deprenyl treated beagles are significantly more successful in terminating the secretion of corticosterone than are control dogs, thus returning to basal levels of circulating glucocorticoids more quickly. The dogs are subsequently sacrificed and numbers of pyramidal cells in region CA1 of hippocampus, a region especially vulnerable to aging and stress, are quantified. L-deprenyl treated dogs will maintain significantly higher numbers of these neurons than do controls, indicating less age- or stress-induced degeneration in this cognitively important brain region. The dose level is 0.1 mg/kg to 5 mg/kg for a time of months to years, with dosing being from a composition comprising tablets, and given orally. The same frequency of dosing of from one to five times weekly applies here as well as to the treatments later discussed. This test relates to adrenal function.

By slowing the aging process, L-deprenyl will also slow the decline in immune function as determined in several ways. Beagles four to seven years of age are treated with L-deprenyl or vehicle for two to five years. Splenocytes from the dogs are mitogenically challenged and proliferative capacity of the cells are measured. Cells from the L-deprenyl treated dogs display significantly higher levels of proliferation than do cells of control dogs. Further, cells from L-deprenyl treated dogs display both an increase in ability to proliferate in response to IL2 and an increase in secretion of IL2 in response to various antigens. Also, the proportions of T-cells capable of expressing IL2 receptors in response to antigen are significantly higher in L-deprenyl treated than in control dogs. The dose level is 0.1 mg/kg to 5 mg/kg for a time of months to years, with dosing being from a composition comprising tablets, and given orally. This test relates to immune system.

Male and female beagles are administered L-deprenyl beginning at four to seven years of age. Two to five years later, T3 and T4 levels are found to be higher in treated and control males and females. As well, beagles of both sexes are found to have maintained higher levels of TRF, than are control dogs thus approximating T3 and T4 levels of younger beagles. The dose level is 0.1 mg/kg to 5 mg/kg for a time of months to years with dosing being from a composition comprising tablets, and given orally. This test pertains to maintenance of thyroid functions.

It can be seen from the above examples that L-deprenyl when dosed as described herein is effective for use in mammals to retard deterioration of renal function, to retard deterioration of the cognitive process, to retard deterioration of exploratory behavior, to retard weight loss, to maintain normal thyroid function, normal adrenal function, normal immune system function, and to maintain normal body composition.

## Claims

1. The use of the compound L-deprenyl, or a pharmaceutically acceptable form thereof, for the manufacture of a medicament for retarding the normal age-dependent deterioration of the cognitive process of dogs.

2. The use of the compound as claimed in claim 1, characterized in that the L-deprenyl is the levorotary optical isomer.

3. The use of the compound as claimed in claim 1 or 2, characterised in that the L-deprenyl is the hydrochloride addition salt form thereof.

4. The use of the compound as claimed in claim 1, 2 or 3, characterised in that L-deprenyl or a pharmaceutically acceptable form thereof is used at levels from 0.1 mg/kg of body weight to 5.0 mg/kg of body weight.

5. The use of the compound L-deprenyl, or a pharmaceutically acceptable form thereof, for manufacture of a medicament for retarding the age-dependent deterioration of exploratory behaviour of dogs.

6. The use of the compound as claimed in claim 5, characterised in that the L-deprenyl is the levorotory optical isomer.

7. The use of the compound as claimed in claim 5 or 6, characterised in that the L-deprenyl is the hydrochloride addition salt form thereof.

8. The use of the compound as claimed in claim 5, 6 or 7, characterised in that L-deprenyl or a pharmaceutically acceptable form thereof is used at levels from 0.1 mg/kg of body weight to 5.0 mg/kg of body weight.

## Patentansprüche

1. Die Verwendung der Verbindung L-Deprenyl, oder einer pharmazeutisch annehmbaren Form derselben, für die Herstellung eines Medikaments zur Verzögerung der normalen altersbedingten Zerstörung der kognitiven Vorgänge von Hunden.

2. Die Verwendung der Verbindung nach Anspruch 1, dadurch gekennzeichnet, daß das L-Deprenyl das linksdrehende optische Isomer ist.

3. Die Verwendung der Verbindung nach Anspruch 1 oder 2, dadurch gekennzeichnet, daß das L-Deprenyl das Hydrochlorid-Additionssalz davon ist.

4. Die Verwendung der Verbindung nach Anspruch 1, 2 oder 3, dadurch gekennzeichnet, daß das L-Deprenyl, oder eine pharmazeutisch annehmbare Form desselben, in Dosen von 0,1 mg / kg Körpergewicht bis 5,0 mg / kg Körpergewicht verwendet wird.

5. Die Verwendung der Verbindung L-Deprenyl, oder einer pharmazeutisch annehmbaren Form derselben, für die Herstellung eines Medikamente zur Verzögerung der normalen altersbedingten Zerstörung des Suchverhaltens von Hunden.

6. Die Verwendung der Verbindung nach Anspruch 5, dadurch gekennzeichnet, daß das L-Deprenyl das linksdrehende optische Isomer ist.

7. Die Verwendung der Verbindung nach Anspruch 5 oder 6, dadurch gekennzeichnet, daß das L-Deprenyl das Hydrochlorid-Additionssalz davon ist.

8. Die Verwendung der Verbindung nach Anspruch 5, 6 oder 7, dadurch gekennzeichnet, daß das L-Deprenyl, oder eine pharmazeutisch annehmbare Form desselben, in Dosen von 0,1 mg / kg Körpergewicht bis 5,0 mg / kg Körpergewicht verwendet wird.

## Revendications

1. Utilisation du composé L-déprényl, ou d'une forme pharmaceutiquement acceptable de celui-ci, pour préparer un médicament pour retarder la dégradation normale fonction de l'âge du processus cognitif des chiens.

2. Utilisation du composé selon la revendication 1, caractérisée en ce que le L-déprényl est l'isomère optique lévogyre.

3. Utilisation du composé selon la revendication 1 ou la revendication 2, caractérisée en ce que le L-déprényl se présente sous sa forme de sel d'addition, chlorhydrate.

4. Utilisation du composé selon l'une des revendications 1, 2 ou 3, caractérisée en ce que le L-déprényl ou une de ses formes pharmaceutiquement acceptables est utilisé à des taux allant de 0,1 mg/kg de poids corporel à 5,0 mg/kg de poids corporel.

5. Utilisation du composé L-déprényl ou l'une de ses formes pharmaceutiquement acceptables, pour préparer un médicament pour retarder la dégradation fonction de l'âge du comportement d'exploration des chiens.

6. Utilisation du composé selon la revendication 5, caractérisée en ce que le L-déprényl est l'isomère optique lévogyre.

7. Utilisation du composé selon la revendication 5 ou la revendication 6, caractérisée en ce que le L-déprényl se présente sous sa forme de sel d'addition, chlorhydrate.

8. Utilisation du composé selon l'une des revendications 5, 6 ou 7, caractérisée en ce que L-déprényl ou l'une de ses formes pharmaceutiquement acceptables est utilisé à des taux de 0,1 mg/kg de poids corporel jusqu'à 5,0 mg/kg de poids corporel.
